# EUROPÄISCHE PATENTANMELDUNG

(11) **EP 3 721 918 A1**
(43) Veröffentlichungstag der Anmeldung: **14.10.2020**
(21) Anmeldenummer: 19168516.3
(22) Anmeldetag: 10.04.2019
(51) Int. Cl.: A61M 1/34, A61M 1/36, A61M 1/16

(54) **HÄMODIALYSEANORDNUNG**

(71) Anmelder: D.Med Consulting GmbH, 20359 Hamburg (DE)
(72) Erfinder: BREUCH, Gerd, 20253 Hamburg (DE)
(74) Vertreter: terpatent Patentanwälte ter Smitten Eberlein-Van Hoof Rütten Daubert

(57) **Zusammenfassung**

Die Erfindung bezieht sich auf eine Hämodialyseanordnung (10) mit einem Hämodialysegerät (20) und einem einen Dialysator (62) aufweisendes Schlauchset (60), das über eigene Fluidkupplungen (31,33,35) und korrespondierende geräteseitige Fluidkupplungen (30,32,34) mit dem Hämodialysegerät (20) fluidisch verbunden ist, wobei das Hämodialysegerät (20) zwei Dialysator-Fluidkupplungen (30,32) aufweist, an denen zwei korrespondierende Fluidkupplungen (31,33) des Schlauchset-seitigen Dialysators (62) angeschlossen sind, und eine Füllflüssigkeits-Fluidkupplung (34) aufweist, die durch eine geräteseitige Füllflüssigkeits-Pumpe (28) mit einer Füllflüssigkeit (44')gespeist werden kann, wobei das Schlauchset (60) eine Füllflüssigkeits-Fluidkupplung (35) aufweist, die an die geräteseitigen Füllflüssigkeits-Fluidkupplung (34) angeschlossen ist, und eine sich daran anschließende Füllflüssigkeits-Leitung (80) aufweist, die über einen fluidischen Füllflüssigkeits-Einleitanschluss (82) in eine blutführende Leitung (72) des Schlauchsets (60) oder den Dialysator (62) einmündet, wobei das Hämodialysegerät (20) eine Gerätesteuerung (50) aufweist, die ein Spülmodul (52) aufweist, das derart ausgebildet ist, dass während der Blutzirkulation in dem Schlauchset (60) nach einem Füllflüssigkeits-Stillstandsintervall von höchstens 120 Minuten nach dem letzten Fluss von Füllflüssigkeit durch die Füllflüssigkeits-Leitung (80) des Schlauchsets (60) automatisch eine Zwangsspülung der gesamten Füllflüssigkeits-Leitung (80) erfolgt.

## Beschreibung

Die Erfindung bezieht sich auf eine Hämodialyseanordnung bestehend aus einem Hämodialysegerät und einem einen Dialysator aufweisenden geräteexternen Schlauchset.

Das Schlauchset weist Fluidkupplungen auf, die mit korrespondierenden geräteseitigen Fluidkupplungen zusammengesteckt sind, so dass das Schlauchset mit dem Hämodialysegerät fluidisch verbunden ist. Das Schlauchset weist einen extrakorporalen Blutkreis auf, durch den während der eigentlichen Blutreinigungs -Behandlung bzw. -Sitzung Patientenblut durch eine arterielle blutführende Leitung, den Dialysator und eine venöse blutführende Leitung fließt.

Aus DE 10 2016 117 725 A1 ist eine derartige Hämodialyseanordnung bekannt. Die Hämodialyseanordnung weist geräteseitig eine Füllflüssigkeits-Fluidkupplung auf, die durch eine Füllflüssigkeits-Pumpe mit einer Füllflüssigkeit, auch Substituierflüssigkeit genannt, aus einem Füllflüssigkeits-Tank gespeist wird bzw. werden kann. Die Füllflüssigkeit dient unter anderem dazu, nach der Beendigung der eigentlichen Blutreinigungs-Behandlung, während der in dem extrakorporalen Blutkreis des Schlauchsets Patientenblut fließt, das Patientenblut aus den blutführenden Leitungen und dem Dialysator zurück zum Patienten zu schieben, damit dieses nicht verloren geht. Das Zurückschieben des Patientenblutes zum Patienten, auch Blut-Reinfusion genannt, erfolgt in der Regel dadurch, dass die blutführenden Leitungen und der Dialysator mit Füllflüssigkeit gefüllt werden. In der Regel wird während der eigentlichen Blutreinigungs-Behandlung, die im Allgemeinen mehrere Stunden dauert, keine Füllflüssigkeit in die blutführenden Leitungen es Schlauchsets eingespeist.

Aufgabe der Erfindung vor diesem Hintergrund ist es, eine Hämodialyseanordnung mit einer betriebssicheren und effektiven Handhabung der Blut-Reinfusion zu schaffen.

Diese Aufgabe wird erfindungsgemäß gelöst mit einer Hämodialyseanordnung mit den Merkmalen des Anspruchs 1.

Die Hämodialyseanordnung besteht im Wesentlichen aus einem Hämodialysegerät und einem fluidisch mit dem Hämodialysegerät verbundenen oder verbindbaren Schlauchset, das außerhalb des Hämodialysegeräts angeordnet ist und in der Regel als Einmalgebrauchs-Artikel ausgebildet ist. Das Schlauchset weist einen Dialysator, eine arterielle blutführende Leitung, eine venöse blutführende Leitung und eine Füllflüssigkeits-Leitung auf, die über einen fluidischen Füllflüssigkeits-Einleitanschluss in eine der beiden blutführenden Leitungen oder in den Dialysator mündet.

Das Schlauchset ist über mehrere Fluidkupplungen fluidisch mit dem Hämodialysegerät verbunden. Im Einzelnen weist das Schlauchset zwei Dialysator-Fluidkupplungen auf, die mit korrespondierenden Dialysator-Fluidkupplungen des Dialysegeräts fluidisch verbunden bzw. verbindbar sind. Ferner weist das Schlauchset an dem freien Ende der Füllflüssigkeits-Leitung eine Füllflüssigkeits-Fluidkupplung auf, die mit einer korrespondierenden Füllflüssigkeits-Fluidkupplung des Hämodialysegeräts zusammengesteckt bzw. zusammensteckbar ist.

Geräteseitig ist eine Füllflüssigkeits-Pumpe vorgesehen, die eine Füllflüssigkeit aus einem Füllflüssigkeits-Tank bzw. einer Füllflüssigkeits-Quelle zu dem Füllflüssigkeits-Einleitanschluss pumpt. Die Füllflüssigkeit kann jede Flüssigkeit sein, die dazu geeignet ist, das Patientenblut zu verdünnen. Die Füllflüssigkeit kann beispielsweise auch die Dialysierflüssigkeit sein, wenn diese hierzu geeignet und entsprechend keimfrei ist. Die Füllflüssigkeit kann jedoch auch eine separate Flüssigkeit sein, die nicht der Dialysierflüssigkeit entspricht.

Die Füllflüssigkeits-Leitung verbindet die Füllflüssigkeits-Fluidkupplung fluidisch mit dem Füllflüssigkeits-Einleitanschluss, durch den die Füllflüssigkeit in eine blutführende Leitung des Schlauchsets eingeleitet werden kann. Der Füllflüssigkeits-Einleitanschluss kann im Verlauf der venösen Blutleitung, im Verlauf der arteriellen Blutleitung oder auf der Blutseite des Dialysators vorgesehen sein. Besonders bevorzugt ist der Füllflüssigkeits-Einleitanschluss im Verlauf der arteriellen blutführenden Leitung des Schlauchsets vorgesehen.

Das Hämodialysegerät weist eine Gerätesteuerung auf, die wiederum ein Spülmodul aufweist, das die regelmäßige Spülung der Füllflüssigkeits-Leitung des Schlauchsets steuert. Das Spülmodul ist hierzu derart ausgebildet, dass während der Blutbehandlungs-Sitzung, während der in dem extrakorporalen Blutkreis des Schlauchsets kontinuierlich Patientenblut fließt, nach einem Füllflüssigkeits-Stillstandsintervall von höchstens 120 Minuten nach dem letzten Fluss von Füllflüssigkeit durch die Füllflüssigkeits-Leitung automatisch eine Zwangsspülung der gesamten Füllflüssigkeits-Leitung mit Füllflüssigkeit erfolgt.

Eine Blutbehandlungs-Sitzung, in der das Patientenblut durch die blutführenden Leitungen des Schlauchset zirkuliert, dauert in der Regel mehrere Stunden. Die Temperatur der Flüssigkeiten in der Hämodialyseanordnung beträgt in der Regel ungefähr 37 °C und die Umgebungstemperatur beträgt 20 °C und mehr. Während der Blutbehandlungs-Sitzung erfolgt in der Regel keine therapeutisch bedingte Auffüllung des Blutkreises mit Füllflüssigkeit, sodass die Füllflüssigkeit in der Füllflüssigkeits-Leitung während der gesamten Blutbehandlungs-Sitzung stillsteht. Bei den relativ hohen Temperaturen kann daher davon ausgegangen werden, dass sich die Anzahl bestimmter kritischer Keime der in dem in der Füllflüssigkeits-Leitung stillstehenden Flüssigkeitssäule ungefähr alle 20 Minuten verdoppelt. Nach einer mehrstündigen Blutbehandlungs-Sitzung müsste daher die Flüssigkeitssäule in der Füllflüssigkeits-Leitung zunächst entsorgt werden, um anschließend frische und ausreichend keimfreie Füllflüssigkeit zur Reinfusion des Patientenbluts in den Patienten in den Schlauchset-Blutkreis einleiten zu können.

Gemäß der Erfindung ist nunmehr vorgesehen, dass mindestens alle 120 Minuten das gesamte Füllflüssigkeits-Volumen der Füllflüssigkeits-Leitung in den Blutkreis des Schlauchsets eingeleitet wird, um die gesamte Füllflüssigkeits-Leitung auf diese Weise immer wieder mit frischer und annähernd keimfreier Füllflüssigkeit zu füllen. Nach dem Abschluss der eigentlichen Blutbehandlungs- Sitzung kann daher sofort mit der Reinfusion des Patientenbluts aus den blutführenden Leitungen des Schlauchsets begonnen werden, indem die Füllflüssigkeit aus der Füllflüssigkeits-Leitung sofort und unmittelbar in die blutführende Leitung eingeleitet werden kann, da diese maximal für die Dauer des voreingestellten Stillstandsintervalls in der Füllleitung stand. Auf diese Weise wird sichergestellt, dass die Füllflüssigkeits-Leitung nicht verkeimt ist und daher vor der Blut-Reinfusion nicht desinfiziert werden muss.

Vorzugsweise ist vorgesehen, dass das längste zulässige Füllflüssigkeits-Stillstandsintervall höchstens 60 Minuten, und ganz bevorzugt höchstens 40 Minuten beträgt. Bei einem Stillstandsintervall von unter 60 bzw. unter 40 Minuten ist sichergestellt, dass keine bedenkliche Verkeimung der Füllflüssigkeit in der Füllflüssigkeits-Leitung vorkommen kann.

Gemäß einer bevorzugten Ausgestaltung ist vorgesehen, dass die extrakorporale Füllflüssigkeits-Leitung ein Gesamt-Fluidvolumen von höchstens 100 ml aufweist. Das in dem Spülmodul digital hinterlegte Zwangsspülungs-Volumen für eine Zwangsspülung orientiert sich am Gesamt-Fluidvolumen der Füllflüssigkeits-Leitung. Die Füllflüssigkeits-Leitung ist relativ kurz und dadurch kleinvolumig, wodurch auch das erforderliche Zwangsspülungs-Volumen einzeln wie auch in der Summe aller Zwangsspülungen möglichst klein gehalten werden kann.

Das Zwangsspülungs-Volumen einer Zwangsspülung beträgt höchstens das Doppelte des Gesamt-Fluidvolumens der Füllflüssigkeits-Leitung. In das Zwangsspülungs-Volumen geht auch noch das Volumen der geräteseitigen Zuführung der Füllflüssigkeit zu der geräteseitigen Füllflüssigkeits-Fluidkupplung mit ein. Dieses kann und sollte konstruktiv ebenfalls möglichst kleinvolumig ausfallen. Durch die Kleinvolumigkeit der Füllflüssigkeits-Leitung wird sichergestellt, dass durch die mehreren Zwangsspülungen über die gesamte Dauer einer Blutbehandlungs-Sitzung keine allzu große Füllflüssigkeitsmenge in den Blutkreis eingespeist wird.

Besonders bevorzugt ist vorgesehen, dass die Füllflüssigkeits-Pumpe innerhalb des Hämodialysegeräts vorgesehen ist. Insbesondere ist die Füllflüssigkeits-Pumpe keine peristaltische Pumpe, sondern beispielsweise als Kolbenpumpe ausgebildet, deren flüssigkeitsgefülltes Volumen bei Stillstand der Pumpe praktisch gleich Null sein kann. Auf diese Weise wird das Zwangsspülungs-Volumen möglichst klein gehalten.

Vorzugsweise ist vorgesehen, dass im Verlauf der Füllflüssigkeits-Leitung keine aktuierte Schlauchklemme vorgesehen ist. Vielmehr kann vorgesehen sein, dass im Verlauf der Füllflüssigkeits-Leitung ein einfaches mechanisches und antriebsloses Rückschlagventil angeordnet ist, durch das ein Einfließen von Blut aus der betreffenden blutführenden Leitung, die den Füllflüssigkeits-Einleitanschluss aufweist, in die Füllflüssigkeit-Leitung auf einfache Weise ausgeschlossen werden kann.

Gemäß einer bevorzugten Ausgestaltung weist die Gerätesteuerung ein Bilanziermodul auf, dass das in den Blutkreis eingespeiste Zwangsspülungs-Volumen in die Volumen-Bilanzierung mit einbezieht. Unter der Bilanzierung wird vorliegend die Erfassung aller FlüssigkeitsVolumina verstanden, die in den Blutkreis des Schlauchset eingespeist bzw. diesem entnommen werden. In der Regel wird beispielsweise angestrebt, dass das gesamte Blutvolumen in dem gesamten Blutkreis, also die Summe der Blutvolumina in dem extrakorporalen Blutleitungen des Schlauchsets und in dem Patienten, möglichst gleichbleibt oder sogar verringert wird. In jedem Fall muss das gesamte Blutvolumen gezielt überwacht und gezielt gesteuert werden. Da durch die regelmäßige Spülung der Füllflüssigkeits-Leitung zusätzliches Flüssigkeitsvolumen in den Blutkreis eingespeist wird, wird dieses durch das Bilanziermodul in die fluidische Steuerung der Blutreinigungs-Sitzung mit einbezogen.

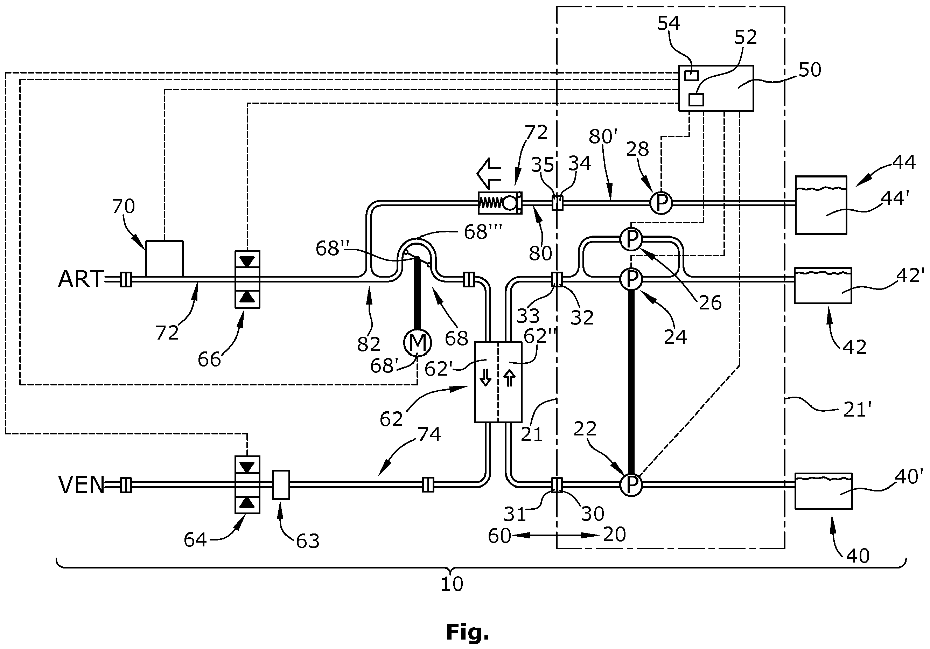

Im Folgenden wird ein Ausführungsbeispiel der Erfindung anhand der Zeichnung näher erläutert.

Die Zeichnung zeigt schematisch eine Hämodialyseanordnung mit einem Hämodialysegerät und einem einen Dialysator aufweisenden Schlauchset.

Die Figur zeigt eine Hämodialyseanordnung 10, die im Wesentlichen von einem Hämodialysegerät 20 und einem externen Schlauchset 60 gebildet ist, das fluidisch an das Hämodialysegerät 20 angeschlossen ist. Das Hämodialysegerät 20 weist einen Dialysierflüssigkeits-Tank 40 mit einer Dialysierflüssigkeit 40', einen Abfalltank 42 mit Abfallflüssigkeit 42' sowie einen Füllflüssigkeits-Tank 44 mit einer Füllflüssigkeit 44' auf. Die Tanks 40,42,44 sind außerhalb eines Gerätegehäuses 21' des Hämodialysegeräts 20 angeordnet.

Ferner weist das Hämodialysegerät 20 innerhalb seines Gerätegehäuses 21' mehrere Flüssigkeits-Pumpen 22,24,26,28 auf, nämlich eine Dialysierflüssigkeits-Pumpe 22, die Dialysierflüssigkeit 40' aus dem Dialysierflüssigkeits-Tank 40 zu einer Dialysierflüssigkeits-Kupplung 30 pumpt, eine Abfallflüssigkeits-Pumpe 24, die die Dialysat-Flüssigkeit von einer Abfallflüssigkeits-Kupplung 32 zu dem Abfalltank 42 pumpt, eine Ultrafiltrationspumpe 26, die fluidisch parallel zu der Abfallflüssigkeits-Pumpe 24 angeordnet ist, und eine Füllflüssigkeits-Pumpe 28, die Füllflüssigkeit 44' aus dem Füllflüssigkeits-Tank 44 zu einer Füllflüssigkeits-Kupplung 34 pumpt. Die drei vorgenannten Flüssigkeitskupplungen 30,32,34 sind an einer Bedientafel 21 angeordnet, die einen Teil des Gerätegehäuses 21' bildet, und sind von der Geräteaußenseite her zugänglich.

Die Dialysierflüssigkeits-Pumpe 22 und die Abfallflüssigkeits-Pumpe 24 sind mechanisch oder elektronisch zu einer Bilanzierpumpe zusammengekoppelt, sodass die Pumpvolumina der beiden vorgenannten Pumpen 22,24 im zeitlichen Mittel stets exakt gleich sind.

Innerhalb des Gerätegehäuses 21' ist ferner eine elektronische und programmierte Gerätesteuerung 50 angeordnet, die ein Spülmodul 52 und ein Bilanziermodul 54 aufweist. Das Spülmodul 52 und das Bilanziermodul 54 können in Form von elektronischer Hardware, können jedoch auch in Form eines Computerprogramms oder einer Mischung aus beidem ausgebildet sein.

Ferner weist das Hämodialysegerät 20 eine peristaltische Blutpumpe 68 auf, von der das Pumpwerk 68" und der elektrische Pumpen-Antriebsmotor 68' dem Hämodialysegerät 20 zugeordnet sind. Der Antriebsmotor 68' ist innerhalb des Gerätegehäuses 21' angeordnet und das Pumpwerk 68" ist an der Bedientafel 21 außenseitig angeordnet, sodass es von außen zugänglich ist und der venöse Blutleitungs-Schlauch in das Pumpwerk 68" eingelegt werden kann.

Das Schlauchset 60 ist vollständig außerhalb des Gerätegehäuses vorgesehen und besteht im Wesentlichen aus einer arteriellen blutführenden Leitung 72, einer venösen blutführenden Leitung 74, einem Hämodialyse-Dialysator 62 und einer Füllflüssigkeits-Leitung 80. Das Schlauchset 60 ist für den Einmalgebrauch ausgelegt und konzipiert.

Der Dialysator 62 weist eine Blutkammer 62' sowie eine Dialysierflüssigkeits-Kammer 62" auf, die durch eine teildurchlässige Membran voneinander getrennt sind. Die Dialysierflüssigkeits-Kammer 62" ist über eine Dialysierflüssigkeits-Kupplung 31 mit der korrespondierenden Dialysierflüssigkeits-Kupplung 30 des Hämodialysegeräts 20 fluidisch verbunden und ist über eine Abfallflüssigkeits-Kupplung 33 mit der geräteseitigen Abfallflüssigkeit-Kupplung 32 fluidisch verbunden.

Die arterielle Leitung 72 beginnt am arteriellen Patientenzugang ART und mündet in die Blutkammer 62' des Dialysators 62, von wo aus die venöse Leitung 74 zurück zum venösen Patientenzugang VEN führt. Zwischen dem arteriellen Patientenzugang ART und der Blutkammer 62' ist im Verlauf der arteriellen blutführenden Leitung 72 ein Luftsensor 70, eine ansteuerbare Schlauchklemme 66, ein y-artiger Füllflüssigkeits-Einleitanschluss 82 und die in das Pumpwerk 68' eingelegte Pumpen-Leitungsschleife 68"' vorgesehen. Der Füllflüssigkeits-Einleitanschluss 82 ist fluidisch zwischen der arteriellen Schlauchklemme 66 und der Blutpumpe 68 angeordnet. Zwischen der Blutkammer 62' und dem venösen Patientenzugang VEN ist im Verlauf der venösen blutführenden Leitung 74 ein Luftblasen-Fänger 63 sowie eine venöse Blutklemme 64 vorgesehen.

Die Füllflüssigkeits-Leitung 80 ist möglichst kurz und kleinvolumig ausgebildet. Tatsächlich beträgt das Gesamt-Fluidvolumen der Füllflüssigkeits-Leitung 80 weniger als 20 ml, insbesondere weniger als 10 ml. Im Verlauf der Füllflüssigkeit-Leitung 80 ist ein mechanisches Rückschlagventil 72 angeordnet, das einen Fluss in Richtung der Füllflüssigkeits-Kupplung 35 blockiert und nur in entgegengesetzter Richtung zulässt. Im Verlauf der Füllflüssigkeits-Leitung 80 ist keine schaltbare Schlauchklemme vorgesehen.

Eine Blutbehandlungs-Sitzung mit dem Einmal-Schlauchset 60 dauert mehrere Stunden, in der Regel 3-4 Stunden. Während der Blutbehandlungs-Sitzung wird den blutführenden Leitungen 72,74, die zusammen mit dem Dialysator 62 den extrakorporalen Blutkreis definieren, in der Regel keine Füllflüssigkeit aus therapeutischen Gründen hinzugefügt. Vielmehr wird gegebenenfalls dem extrakorporalen Blutkreis über eine entsprechende Steuerung der Ultrafiltrationspumpe 26 Flüssigkeitsvolumen entzogen. Nach der mehrstündigen Blutbehandlungs-Sitzung wird das in dem extrakorporalen Blutkreis befindliche Patientenblut zum Patienten zurück gepumpt, indem durch die Füllflüssigkeit-Pumpe 28 Füllflüssigkeit 44' in den extrakorporalen Blutkreis gepumpt wird. Dieser Vorgang wird auch Blut-Reinfusion genannt. Hierbei wird die Blutpumpe 68 derart durch die Gerätesteuerung 50 gesteuert, dass ein Teil der über den Einleitanschluss 82 in die arterielle Leitung 72 einfließenden Füllflüssigkeit zum arteriellen Patientenzugang ART und ein anderer größerer Teil in Richtung des venösen Patientenzugangs VEN fließt, bis das Patientenblut aus dem extrakorporalen Blutkreis annähernd vollständig zum Patienten zurückgegeben ist.

Während der gesamten Dauer der Blutbehandlungs-Sitzung sorgt das Spülmodul 52 dafür, dass nach jeweils einem Füllflüssigkeits-Stillstandsintervall von beispielsweise 40 oder 20 Minuten nach dem letzten Fluss von Füllflüssigkeit durch die Füllflüssigkeits-Leitung 80 automatisch eine Zwangsspülung der gesamten Schlauchset-Füllflüssigkeits-Leitung 80 sowie der geräteinternen Füllflüssigkeits-Leitung 80' ausgeführt wird. Das Zwangsspülungs-Volumen beträgt hierbei beispielsweise jeweils ca. 100 ml, sodass bei jeder Zwangsspülung die gesamte Füllflüssigkeits-Leitung 80 des Schlauchsets 60 mit frischer Füllflüssigkeit 44' aufgefüllt wird. Sowohl das Füllflüssigkeits-Stillstandsintervall als auch das Zwangsspülungs-Volumen sind in dem Spülmodul 52 gespeichert, und können vom Anwender individuell eingestellt werden.

Durch das regelmäßige Zwangsspülen der Füllflüssigkeits-Leitung 80 des Schlauchsets 60 wird sichergestellt, dass nach dem Ende der eigentlichen Blutbehandlungssitzung keine hochgradig verkeimte Füllflüssigkeit in der Füllflüssigkeits-Leitung 80 steht, die vor dem Start der eigentlichen Reinfusion des Patientenbluts erst entsorgt werden müsste. Auch eine Sterilisierung der Füllflüssigkeits-Leistung 80 vor dem Start der Blut-Reinfusion kann entfallen. Hierdurch wird zum einen ein sehr einfacher fluidische Aufbau des Hämodialysegeräts 20 und des Schlauchset 60 ermöglicht und wird zum anderen eine hohe Sterilität sichergestellt.

## Patentansprüche

1. Hämodialyseanordnung (10) mit einem Hämodialysegerät (20) und einem einen Dialysator (62) aufweisendes Schlauchset (60), das über eigene Fluidkupplungen (31,33,35) und korrespondierende geräteseitige Fluidkupplungen (30,32,34) mit dem Hämodialysegerät (20) fluidisch verbunden ist,
wobei das Hämodialysegerät (20) zwei Dialysator-Fluidkupplungen (30,32) aufweist, an denen zwei korrespondierende Fluidkupplungen (31,33) des Schlauchset-seitigen Dialysators (62) angeschlossen sind, und eine Füllflüssigkeits-Fluidkupplung (34) aufweist, die durch eine geräteseitige Füllflüssigkeits-Pumpe (28) mit einer Füllflüssigkeit (44') gespeist werden kann,
wobei das Schlauchset (60) eine Füllflüssigkeits-Fluidkupplung (35) aufweist, die an die geräteseitigen Füllflüssigkeits-Fluidkupplung (34) angeschlossen ist, und eine sich daran anschließende Füllflüssigkeits-Leitung (80) aufweist, die über einen fluidischen Füllflüssigkeits-Einleitanschluss (82) in eine blutführende Leitung (72) des Schlauchsets (60) oder den Dialysator (62) einmündet,
wobei das Hämodialysegerät (20) eine Gerätesteuerung (50) aufweist, die ein Spülmodul (52) aufweist, das derart ausgebildet ist, dass während der Blutzirkulation in dem Schlauchset (60) nach einem Füllflüssigkeits-Stillstandsintervall von höchstens 120 Minuten nach dem letzten Fluss von Füllflüssigkeit durch die Füllflüssigkeits-Leitung (80) des Schlauchsets (60) automatisch eine Zwangsspülung der gesamten Füllflüssigkeits-Leitung (80) erfolgt.

2. Hämodialyseanordnung (10) nach Anspruch 1, wobei das Spülmodul (52) derart ausgebildet ist, dass das zulässige Füllflüssigkeits-Stillstandsintervall höchstens 60 Minuten beträgt, ganz besonders bevorzugt höchstens 40 Minuten beträgt.

3. Hämodialyseanordnung (10) nach einem der vorangegangenen Ansprüche, wobei die Füllflüssigkeits-Leitung (80) des Schlauchsets (60) ein Gesamt-Fluidvolumen von höchstens 100 ml aufweist und wobei das das Zwangsspülungs-Volumen einer Zwangsspülung höchstens das Doppelte des Gesamt-Fluidvolumens der Füllflüssigkeits-Leitung (80) beträgt.

4. Hämodialyseanordnung (10) nach einem der vorangegangenen Ansprüche, wobei im Verlauf der Füllflüssigkeits-Leitung (80) des Schlauchsets (60) ein Rückschlagventil (72) angeordnet ist.

5. Hämodialyseanordnung (10) nach einem der vorangegangenen Ansprüche, wobei die Füllflüssigkeits-Pumpe (28) innerhalb des Gehäuses des Hämodialysegeräts (20) vorgesehen ist.

6. Hämodialyseanordnung (10) nach einem der vorangegangenen Ansprüche, wobei keine schaltbare Schlauchklemme im Verlauf der Füllflüssigkeits-Leitung (80) des Schlauchsets (60) vorgesehen ist.

7. Hämodialyseanordnung (10) nach einem der vorangegangenen Ansprüche, wobei die Gerätesteuerung (50) ein Bilanziermodul (54) aufweist, das das eingespeiste Zwangsspülungs-Volumen bei der Bilanzierung rechnerisch mit einbezieht.
